# EUROPEAN PATENT APPLICATION

(11) **EP 4 345 095 A1**
(43) Date of publication of application: **03.04.2024**
(21) Application number: 22804101.8
(22) Date of filing: 18.07.2022
(51) Int. Cl.: C07D 215/42, C07D 401/12, C07D 215/36, A61P 19/06, A61P 13/04, A61P 19/02, A61P 13/12

(54) **QUINOLINE MERCAPTOACETATE SULFONAMIDE DERIVATIVE, INTERMEDIATE, PHARMACEUTICAL DERIVATIVE OR FORMULATION, AND PREPARATION METHOD AND USE THEREFOR**

(30) Priority: 19.05.2021 CN 202110542948
(71) Applicant: Jiangsu Chiatai Qingjiang Pharmaceutical Co., Ltd., Huaian, Jiangsu 223005 (CN)
(72) Inventor: CHEN, Jie, Huaian, Jiangsu 223005 (CN); LU, Jun, Huaian, Jiangsu 223005 (CN); XIE, Fujia, Huaian, Jiangsu 223005 (CN); HUANG, Lv, Huaian, Jiangsu 223005 (CN)
(74) Representative: karo IP
(86) International application number: PCT/CN2022/106216
(87) International publication number: WO 2022/242782

(57) **Abstract**

The present application relates to a quinoline mercaptoacetate sulfonamide derivative, an intermediate, a pharmaceutical derivative or a formulation, and a preparation method and use therefor. The structure of the quinoline mercaptoacetate sulfonamide derivative is as represented by the following formula (I). The present application further relates to a preparation method for a compound containing a structure as represented by the following formula. Experiments show that the compound provided in the present invention has a good inhibitory effect on URAT1 transport uric acid in HEK293 transfected cells, and that the compound has good application prospects in the treatment of hyperuricemia or gout.

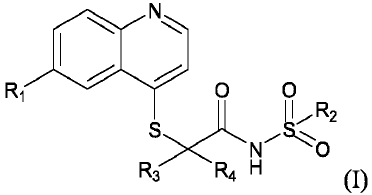

## Description

### TECHNICAL FIELD

The present application belongs to the field of medicines, and relates to synthesis and use of quinoline mercaptoacetate sulfonamide compounds. The present application further relates to use of the compounds and compositions in resisting gout.

### BACKGROUND

Gout is a kind of metabolic disease in which urate is deposited in joints, cartilage, kidney and the like due to blood uric acid level rise caused by purine metabolic disorder or uric acid excretion reduction *in vivo.* It is clinically manifested by recurrent acute and chronic arthritis and soft tissue injury. In addition, hyperuricemia or gout is also closely related to the occurrence of diseases such as hypertension, hyperlipidemia, and atherosclerosis.

The pathogenesis of gout includes two stages: 1, uric acid, an end product of purine metabolism *in vivo*, exists in a form of urate under physiological conditions. When its concentration in the blood exceeds a dissolution threshold (408 µmol/L or 6.8 mg /dL), it may separate out a precipitate to form monosodium urate (MSU) crystals, which are deposited in joints and surrounding tissues; 2, the stimulation of joints and tissues by MSU crystals triggers an immune response and leads to spontaneous inflammation. It can be said that gout is an inflammatory and immune disease caused by metabolic diseases. The main clinical symptoms of people with gout include: increased serum uric acid concentration (i.e., hyperuricemia); joint redness and swelling; recurrent acute and chronic arthritis; long-term accumulation and deposition of MSU in and around joints to form tophi, which in severe cases may cause joint deformities and even disabilities of a patient; renal function damage, involving glomerulopathy, tubulopathy and interstitial back inflammation, and even renal failure and uric acid kidney stones.

The drug treatment methods of gout mainly include: one is to reduce the production of uric acid *in vivo.* That is, a xanthine oxidase inhibitor (XOIS) is adopted, which is the oldest anti-gout drug used for inhibiting xanthine oxidase to prevent xanthine and hypoxanthine from oxidizing to produce urate and hydrogen peroxide. Now it is still used as a first-line drug to reduce uric acid in gout treatment guidelines in many countries, and the main drugs are allopurinol, febuxostat and topistat. The other is drugs that promote uric acid excretion *in vivo.* These drugs may selectively inhibit organic anion transporters (OATs) expressed in renal proximal tubule cells, such as urate transporter 1 (URAT1) and glucose transporter 9 (GLUT9), thus promoting uric acid excretion. These drugs are the mainstream research and development direction of gout treatment drugs, such as Verinurad (clinical stage) and probenecid, benzbromarone, lesinurad (marketed in the United States in 2015).

Therefore, it is of great significance to research a new anti-gout drug with high efficiency, low toxicity and good efficacy.

### SUMMARY

According to the present application, the technical solution is as follows: the present application aims to target a urate transporter URAT1 and devotes to the design and synthesis of a URAT1 inhibitor that inhibits uric acid reabsorption, and research on blood uric acid activity lowering. On the basis of existing research, structural modifications and improvements are made to examine the impact of different groups on the structure-activity relationship of drugs. The *in vitro* hURAT1 inhibitory activity of the obtained compounds are tested and compared with a control substance. It is expected to discover a potential new URAT1 inhibitor with better biological activity.

The present application provides a quinoline mercaptoacetate sulfonamide derivative and a preparation method thereof, and also provides an activity screening result and use of the compound as an anti-gout drug.
I. In a first aspect, the present application provides a quinoline mercaptoacetate sulfonamide derivative, which has a structure as represented by the following formula:

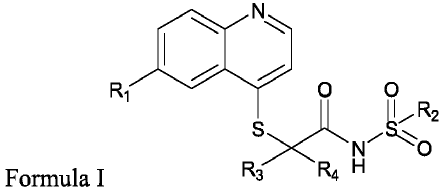

R₁ and R₂ are each independently selected from hydrogen, halogen, cyano, nitro, substituted or unsubstituted C1-C6 linear alkyl, substituted or unsubstituted C3-C7 cycloalkyl, substituted or unsubstituted C3-C7 heterocycloalkyl, and substituted or unsubstituted C4-C12 heterocyclic aryl; heteroatoms are selected from one or more of oxygen, sulfur, and nitrogen; and substituents are selected from one or more of halogen, cyano, nitro, alkoxy, alkyl, haloalkyl, hydroxyalkyl, alkenyl, alkynyl, cycloalkyl, heterocycloalkyl, aryl, and heterocyclic aryl.

R₃ and R₄ are each independently selected from a hydrogen atom, C1-C6 alkyl or cycloalkyl; or R₃ and R₄ form a C3-C6 ring.

In the present application, the "heteroatom" refers to atoms except the C atom and the H atom in the heterocycloalkyl and/or heterocyclic aryl.

The inventors of the present application found that the compound with the structure as represented by Formula I could achieve the effect equivalent to or even better than that of existing gout drugs. In order to further improve the curative effect, one or more of the following specific embodiments may be selected.

### About R₁:

In one specific embodiment, R₁ is selected from hydrogen, halogen, cyano, nitro, C1-C3 linear alkyl, C1-C4 branched alkyl, C3-C6 cycloalkyl, C3-C4 substituted cycloalkyl, phenyl, substituted phenyl, thiophene, and C5-C6 heterocyclic aryl; the substituent is selected from halogen, cyano, methyl, ethyl, n-propyl, isopropyl, cyclopropyl, and cyclobutyl; the heteroatoms in the heterocyclic aryl are selected from one or more of oxygen, sulfur, and nitrogen.

In one specific embodiment, R₁ is selected from hydrogen, halogen, cyano, nitro, C1-C3 linear alkyl, C1-C4 branched alkyl, and C3-C6 cycloalkyl.

In one specific embodiment, R1 is methyl, fluorine, bromine, trifluoromethyl, etc.

### About R₂:

In one specific embodiment, R₂ is selected from hydrogen, halogen, cyano, nitro, C1-C3 linear alkyl, C1-C4 branched alkyl, C3-C6 cycloalkyl, C3-C4 substituted cycloalkyl, phenyl, substituted phenyl, thiophene, and C5-C6 heterocyclic aryl; the substituent is selected from halogen, cyano, methyl, ethyl, n-propyl, isopropyl, cyclopropyl, and cyclobutyl; the heteroatoms in the heterocyclic aryl are selected from one or more of oxygen, sulfur, and nitrogen.

In one specific embodiment, R₂ is selected from C1-C3 linear alkyl, C1-C4 branched alkyl, C3-C6 cycloalkyl, C3-C4 substituted cycloalkyl, phenyl, substituted phenyl, thiophene, and C5-C6 heterocyclic aryl; the substituent is selected from halogen, cyano, methyl, ethyl, n-propyl, isopropyl, cyclopropyl, and cyclobutyl; and the heteroatoms in the heterocyclic aryl are selected from one or more of oxygen, sulfur, and nitrogen.

In one specific embodiment, R₂ is methyl, ethyl, thiophene, cyclopropyl, etc.

### About R₃ group and R₄ group:

In one specific embodiment, R₃ and R₄ are each independently selected from a hydrogen atom, C1-C3 alkyl or cycloalkyl; or R₃ and R₄ form a C2-C4 ring.

According to one specific embodiment, R₃ and R₄ are each independently selected from a hydrogen atom, methyl, and ethyl.

According to one specific embodiment, R₃ is methyl, and R₄ is methyl.

According to one specific embodiment, one of R₃ and R₄ is methyl, and the other is a hydrogen atom.

According to the present application, various embodiments of R₁, R₂, R₃ and R₄ may be combined with one another. Equivalently, the present application discloses various combinations of various embodiments of R₁, R₂, R₃ and R₄. To save text, they will not be described one by one.

Specifically, the compound with the structure as represented by Formula I includes, but is not limited to, the following specific compounds:

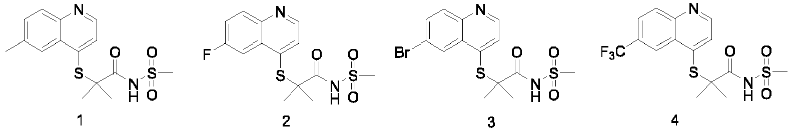

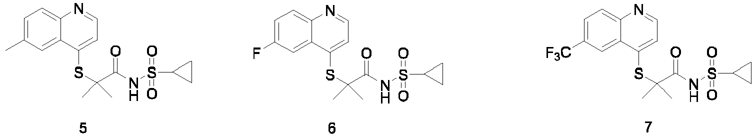

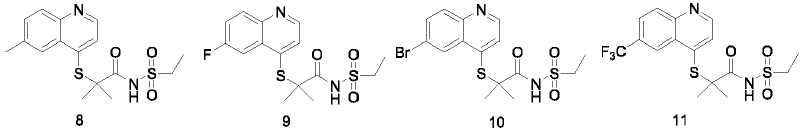

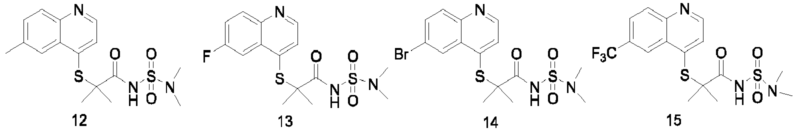

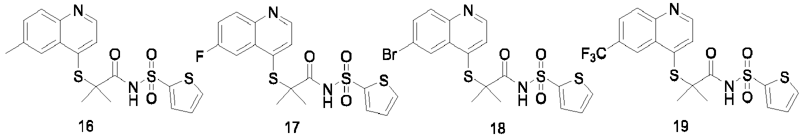

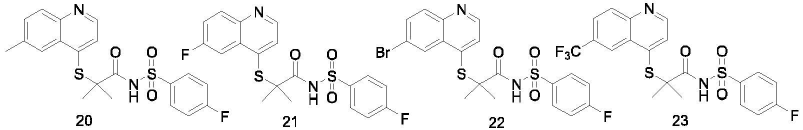

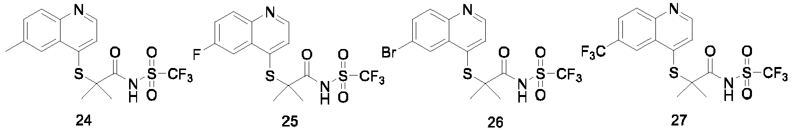

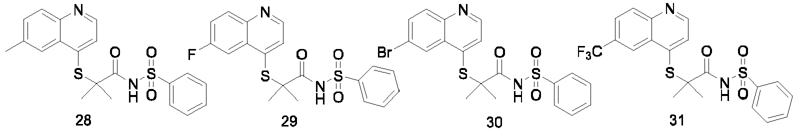

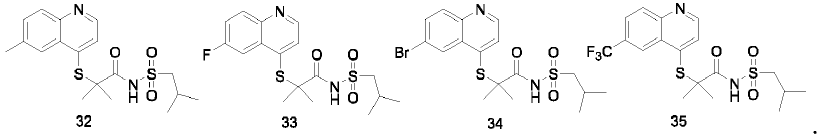

II. In a second aspect, the present application provides an intermediate for preparing the compound of the present application in the first aspect, and the intermediate has the structure as represented by formula II, 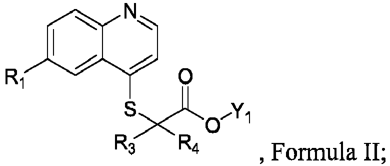
in the structure of the compound as represented by Formula II,
R₁ is R₁ in Formula I, and Y₁ is methyl or ethyl; and
R₃ and R₄ are R₃ and R₄ in Formula I.

### About R₁:

In one specific embodiment, R₁ is selected from hydrogen, halogen, cyano, nitro, C1-C3 linear alkyl, C1-C4 branched alkyl, C3-C6 cycloalkyl, C3-C4 substituted cycloalkyl, phenyl, substituted phenyl, thiophene, and C5-C6 heterocyclic aryl; the substituent is selected from halogen, cyano, methyl, ethyl, n-propyl, isopropyl, cyclopropyl, and cyclobutyl; the heteroatoms in the heterocyclic aryl are selected from one or more of oxygen, sulfur, and nitrogen.

In one specific embodiment, R₁ is selected from hydrogen, halogen, cyano, nitro, C1-C3 linear alkyl, C1-C4 branched alkyl, and C3-C6 cycloalkyl.

In one specific embodiment, R1 is methyl, fluorine, bromine, trifluoromethyl, etc.

### About R₃ group and R₄ group:

In one specific embodiment, R₃ and R₄ are each independently selected from a hydrogen atom, C1-C3 alkyl or cycloalkyl; or R₃ and R₄ form a C2-C4 ring.

According to one specific embodiment, R₃ and R₄ are each independently selected from a hydrogen atom, methyl, and ethyl.

According to one specific embodiment, R₃ is methyl, and R₄ is methyl.

According to one specific embodiment, one of R₃ and R₄ is methyl, and the other is a hydrogen atom.

**III. In a third aspect, the present application provides a method for preparing the compound as represented by Formula II of the present application in the second aspect,** and the method includes:
(1) performing first contact on a compound as represented by Formula III and a sulfide salt in the presence of a first solvent and an inert atmosphere, so as to obtain a compound as represented by Formula IV; and
(2) performing second contact on the compound as represented by Formula IV and a compound as represented by Formula V in the presence of the first solvent and carbonate salt; 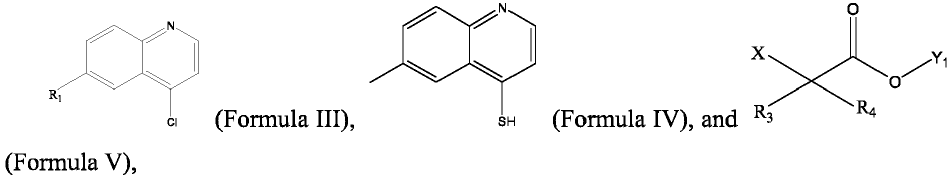
   R₁ is R₁ in Formula I, and Y₁ is methyl or ethyl;
   R₃ and R₄ are R₃ and R₄ in Formula I; and
   X is halogen.

The first contact reaction is performed at 90-105°C for 1.5-2.5 h.

The second contact reaction is performed at room temperature for 6-8 h.

The first solvent is an organic solvent, such as N,N-dimethylformamide or acetonitrile.

The sulfide salt includes, but is not limited to, one or a combination of more of sodium sulfide, potassium sulfide, and magnesium sulfide.

The carbonate salt includes, but is not limited to, one or a combination of more of sodium carbonate, potassium carbonate, and cesium carbonate.

In one specific embodiment, a method for preparing the compound as represented by Formula II includes:
(1) performing first contact reaction on 4-chloro-6 substituted quinoline and sodium sulfide in the presence of N,N-dimethylformamide (DMF) to obtain 4-mercapto-6 substituted quinoline; and
(2) performing second contact reaction on the intermediate compound 4-mercapto-6 substituted quinoline and 2-halogen-fatty acid ethyl ester in the presence of DMF and carbonate salt.

In one specific embodiment, in step (1), the first contact reaction is performed under the protection of inert gas (such as nitrogen gas).

In one specific embodiment, in step (1), the first contact reaction is performed at 90-105°C for 1.5-2.5 h.

In one specific embodiment, in step (1), a mixed solution of ethyl acetate and petroleum ether in a weight ratio of 1: (4-8) is used as a developing agent for thin-layer chromatography to monitor reaction.

In one specific embodiment, the material obtained in the first contact reaction in step (1) is subjected to cooling, extraction, pH value regulation (to 5-6), and solid-liquid separation to obtain the intermediate compound 4-mercapto-6 substituted quinoline.

In one specific embodiment, in step (2), a mixed solution of ethyl acetate and petroleum ether in a weight ratio of 1: (0.8-1.2) is used as a developing agent for thin-layer chromatography to monitor reaction.

In one specific embodiment, the material obtained in the second contact reaction in step (2) is subjected to extraction, washing, drying, concentration and separation to obtain the compound as represented by Formula II.

**IV. In a fourth aspect, the present application provides a method for preparing the compound as represented by Formula I of the present invention in the first aspect, and the method includes: sequentially performing hydrolysis reaction and sulfonamide reaction on the compound as represented by Formula II to obtain the compound as represented by Formula I.**

In one specific embodiment, the specific reaction formula for preparing the compound as represented by Formula I is as follows:

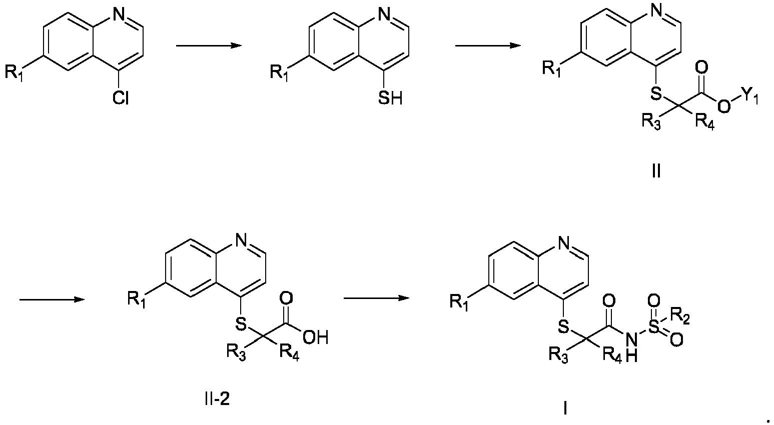

The hydrolysis reaction may be performed according to the conventional mode in the art.

In one specific embodiment, the hydrolysis reaction process includes: performing third contact on a solution containing the compound as represented by Formula II and an alkaline aqueous solution, and performing heating reflux.

In one specific embodiment, in the hydrolysis reaction, a second solvent for dissolving the solution of the compound as represented by Formula II is an organic solvent, such as methanol and ethanol.

In one specific embodiment, in the hydrolysis reaction, the alkaline aqueous solution is a 15-30 wt% NaOH solution.

In one specific embodiment, in the hydrolysis reaction, after the reaction is finished (determined by TLC monitoring), cooling (preferably simultaneous dilution, for example, by adding cold water) is performed, and the pH is regulated to be acidic (for example, pH=1-2).

In one specific embodiment, in the hydrolysis reaction, the material of which the pH is regulated is filtered, washed and dried to obtain the compound with the structure as represented by Formula II-2.

The sulfonamide reaction may be performed according to the conventional mode in the art.

In one specific embodiment, the sulfonamide reaction process includes: in the presence of a second solvent (such as dichloromethane), 1-ethyl-(3-dimethylaminopropyl)carbodiimide hydrochloride (EDC) and 4-dimethylaminopyridine (DMAP), performing a fourth contact reaction on the compound as represented by Formula II-2 and a sulfonamide compound.

In one specific embodiment, in the sulfonamide reaction, the compound as represented by Formula II-2 is mixed with the second solvent, then the EDC, DMAP and sulfonamide compound are added at a temperature of -2°C to 5°C, and the temperature is raised to room temperature (20-30°C) in the reaction.

It is to be noted that the numerical representations such as "first", "second", "third", and "fourth" in the present application are only used for distinguishing different reaction processes, and different forms of existence or use do not represent the difference in order.

**V. In a fifth aspect, the present application provides a pharmaceutical derivative or a formulation of the compound with the structure as represented by Formula I of the present application in the first aspect, and the pharmaceutical derivative or the formulation includes a pharmaceutically acceptable salt, a composition, a solvate, a hydrate, and a pharmaceutically acceptable prodrug.**

**VI. In a sixth aspect, the present application provides a pharmaceutical derivative or a formulation of the compound with the structure as represented by Formula II of the present application in the second aspect, and the pharmaceutical derivative or the formulation includes a pharmaceutically acceptable salt, a composition, a solvate, a hydrate, and a pharmaceutically acceptable prodrug.**

The pharmaceutical derivatives or the formulations of the present application in the fifth and sixth aspects may be obtained by adding one or more pharmaceutically acceptable carriers, auxiliary materials and excipients which are conventional in the art using a conventional preparation method in the art.

The pharmaceutically acceptable salt includes, but is not limited to, for example, Na, K, Li, Mg, Ca, and Zn salts.

The pharmaceutically acceptable prodrug includes, but is not limited to, for example, ester, carbonate ester, enacarbil ester, thiocarbonate ester, N-acyl derivative, N-acyloxy derivative, and amino acid conjugate.

The pharmaceutical derivative or the formulation may contain a carrier, which includes, but is not limited to, for example, mannitol, sorbitol, sodium pyrosulfite, sodium bisulfite, sodium thiosulfate, cysteine hydrochloride, mercaptoacetic acid, methionine, vitamin C, EDTA disodium, EDTA calcium sodium, carbonate, acetate and phosphate salts of monovalent alkali metal or an aqueous solution thereof, hydrochloric acid, acetic acid, sulfuric acid, phosphoric acid, amino acid, sodium chloride, potassium chloride, sodium lactate, xylitol, maltose, glucose, fructose, dextran, glycine, starch, sucrose, lactose, mannitol, a silicon derivative, cellulose and a derivative thereof, alginate, gelatin, polyvinylpyrrolidone, glycerin, Tween 80, agar, calcium carbonate, calcium bicarbonate, a surfactant, polyethylene glycol, cyclodextrin, β-cyclodextrin, a phospholipid material, kaolin, talcum powder, calcium stearate, and magnesium stearate. For example, it is used in a solid orally administered preparation.

The pharmaceutical derivative or the formulation may contain an excipient, which includes, but is not limited to, for example, an adhesive, a filler, a diluent, a tableting agent, a lubricant, a disintegrant, a colorant, a flavoring agent, and a humectant, and the tablet may be coated if necessary. For example, it is used in a solid orally administered preparation.

The pharmaceutical derivative or the formulation may contain the filler, which includes, but is not limited to, for example, cellulose, mannitol, and lactose. For example, it is used in a solid orally administered preparation.

The pharmaceutical derivative or the formulation may contain the disintegrant, which includes, but is not limited to, for example, starch, polyvinylpyrrolidone, and a starch derivative, e.g. sodium starch glycolate. For example, it is used in a solid orally administered preparation.

The pharmaceutical derivative or the formulation may contain the lubricant, which includes, but is not limited to, for example, magnesium stearate. For example, it is used in a solid orally administered preparation.

The pharmaceutical derivative or the formulation may contain the humectant, which includes, but is not limited to, for example, sodium lauryl sulfate. For example, it is used in a solid orally administered preparation.

The pharmaceutical derivative or the formulation may contain a suspending agent, which includes, but is not limited to, for example, sorbitol, syrup, methylcellulose, gelatin, hydroxyethyl cellulose, carboxymethylcellulose, aluminum stearate gel, and hydrogenated edible fat. For example, it is used in a liquid orally administered preparation (e.g., aqueous or oily suspension, solution, emulsion, syrup, or elixir) or in a dry product that may be compounded with water or other suitable carriers prior to use.

The pharmaceutical derivative or the formulation may contain an emulsifier, which includes, but is not limited to, for example, lecithin, sorbitan monooleate, and gum arabic. For example, it is used in a liquid orally administered preparation or in a dry product.

The pharmaceutical derivative or the formulation may contain a non-aqueous carrier (which may include edible oil), which includes, but is not limited to, for example, almond oil, fractionated coconut oil, oily ester such as ester of glycerol, propylene glycol, and ethanol. For example, it is used in a liquid orally administered preparation or in a dry product.

The pharmaceutical derivative or the formulation may contain a preservative, which includes, but is not limited to, for example, methylparaben, propyl p-hydroxybenzoate, and sorbic acid. For example, it is used in a liquid orally administered preparation or in a dry product.

The pharmaceutical derivative or the formulation can contain a sterile carrier, for example, in an injection. According to the carrier and concentration, the compound according to the present application may be suspended or dissolved. The solution is generally prepared by dissolving the compound in the carrier, filtering and disinfecting the carrier before the carrier is put into a proper vial or ampoule, and then sealing the vial or ampoule. The auxiliary materials such as a local anesthetic, a preservative and a buffer agent may also be dissolved in the carrier. In order to improve the stability, the composition may be frozen after being put into the vial, and water is removed under vacuum.

According to the present application, the quinoline mercaptoacetate sulfonamide derivative may be in any medicinal dosage form, and the dosage forms include tablets, sugar-coated tablets, film-coated tablets, enteric-coated tablets, capsules, hard capsules, soft capsules, oral liquid, buccal tablets, granules, electuaries, pills, sprinkles, ointments, pellets, suspensions, powders, solutions, injections, suppositories, ointments, plasters, creams, spray, drops and patches.

According to the present application, the preparation is preferably in an oral dosage form, such as capsules, tablets, oral liquid, granules, pills, sprinkles, electuaries, ointments and the like.

According to the present application, the administration route may be oral administration, parenteral administration or local administration, and preferably oral administration and injection administration. The orally administered preparation suitable for medicine may be tablets, capsules, granules or other liquid preparations suitable for medicine, such as solution, emulsion, and suspending agent. The preferably oral preparation is the tablets, and the tablets may be prepared into a coating, enteric coating, slow release or quantitative release form. A solid oral composition may be prepared by common methods such as mixing, filling, and tabletting. The active substances may be distributed in the whole composition using a large amount of filler by repeated mixing.

In the sixth aspect, the present application provides use of the compound as represented by Formula I and the pharmaceutical derivative or the formulation thereof, and/or the compound as represented by Formula II and the pharmaceutical derivative or the formulation thereof in preparation of a medicine for regulating the level of uric acid and/or treating gout-related indications.

The related indications include, but are not limited to, hyperuricemia, gout, gouty arthritis, inflammatory arthritis, nephropathy, nephrolithiasis, arthritis, urate crystal deposition in joints, urolithiasis, urate crystal deposition in renal parenchyma, gout attack, tophaceous gout, or a combination thereof.

According to the present application, the compound as represented by Formula I or Formula II and the pharmaceutical derivative or the formulation thereof have good URAT1 inhibitory activity, can be used for treatment of gout and hyperuricemia, and provide a new medicinal possibility for clinical treatment of diseases related to URAT1 activity abnormality.

The endpoints of ranges and any values disclosed herein are not limited to the precise range or value, but these ranges or values are to be understood to include values approaching such ranges or values. For numerical ranges, the endpoint values of each range, the endpoint values of each range and individual point values, and the individual point values may be combined with each other to obtain one or more new numerical ranges. These value ranges shall be deemed to be specifically disclosed herein.

### DETAILED DESCRIPTION

The present application will be described in detail by examples below. The examples described in the present application are only some rather than all of the examples of the present application. Based on the examples in the present application, all other examples obtained by a person of ordinary skill in the art without creative efforts shall fall within the scope of protection of the present application.

The following takes the preparation process of the compound as an example.

### Example 1

Synthesis of compound 1: The synthetic route is as follows:

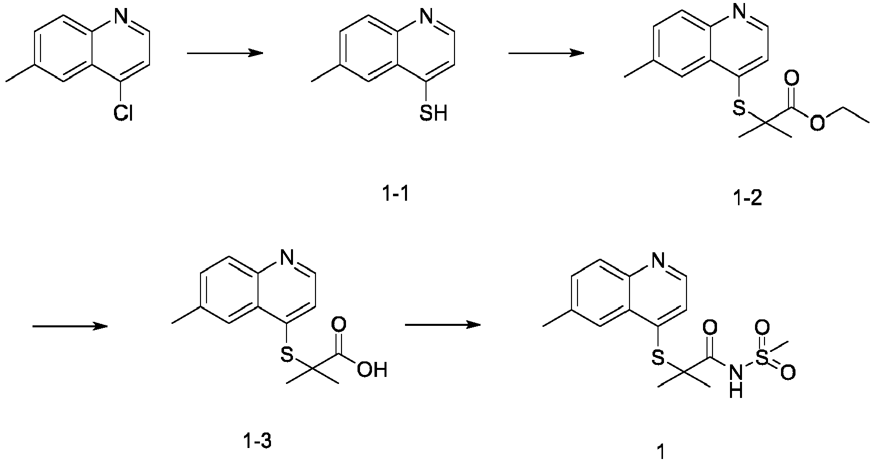

Synthesis of intermediate 1-1: 4-chloro-6-methylquinoline (5.0 g, 28 mmol), sodium sulfide (13.5 g, 56 mmol), and 25 mL of DMF were added into a 100 mL three-neck flask, and stirred at 100°C for 2 h under the protection of nitrogen gas. After cooling to room temperature, the reaction solution was poured into 100 mL of water, and extracted with dichloromethane (50 mL *3), and then an organic phase was removed. The pH of the water phase was regulated to 5-6 with concentrated hydrochloric acid; and stirring was performed for 1 h to separate out a yellow solid. Vacuum filtration was performed, and vacuum drying was performed at 50°C to obtain 4.0 g of a yellow solid, and the yield was 81%.

Synthesis of intermediate 1-2: The intermediate 1-1 (3.3 g, 19 mmol), ethyl bromomethylpropionate (5.5 g, 28 mmol), cesium carbonate (12.3 g, 38 mmol) and 30 mL of DMF were added into a 100 mL three-neck flask, and stirred at room temperature for 2 h. The reaction solution was poured into 100 mL of ice water, and extracted with DCM (50 mL *3), and the organic phase was combined. Then washing was performed with a saturated salt solution (100 mL *2), drying was performed with sodium sulfide, and the organic phase was concentrated. Column chromatography (300-400 meshes, and eluted with petroleum ether) was performed on the crude product to obtain 5.1 g of a colorless oily substance; and the yield was 94%.

Synthesis of intermediate 1-3: The intermediate 1-2 (5 g, 17 mmol), sodium hydroxide (1.38 g, 34 mmol), 30 mL of methanol and 5 mL of water were added into a 100 mL three-neck flask, and stirred at 60°C for 2 h. After the reaction was finished, 20 mL of water was added for dilution, and concentrating was performed to remove methanol. The pH of the water phase was regulated to 1-2 with concentrated hydrochloric acid to obtain a yellow solid, and the yellow solid was subjected to vacuum drying at 50°C to obtain 3.3 g of the crude product. The yield was 74%.

Synthesis of compound 1: The intermediate 1-3 (0.6 g, 2.3 mmol), methyl sulfonamide (0.65 g, 6.8 mmol), HATU (1.1 g, 2.9 mmol), DIPEA (0.9 g, 6.9 mmol) and 7 mL of DMF were added into a reaction bottle, and stirred at room temperature for 24 h. The reaction solution was poured into 50 mL of ice water, extracted with EA (50 mL *3), washed with saturated salt water (50 mL *2), dried over sodium sulfide, and then concentrated to obtain a crude product. High-pressure preparation separation (reversed-phase column C-18, mobile phase: acetonitrile and water) was performed on the crude product. Concentrating was performed to remove acetonitrile. Then freeze-drying was performed to obtain 0.23 g of a white solid. The yield was 30%. Mass spectrum: 340.0 (M+H⁺).

### Example 2

The synthesis method of compound 2 is the same as the synthesis method of compound 1. The synthetic route is as follows:

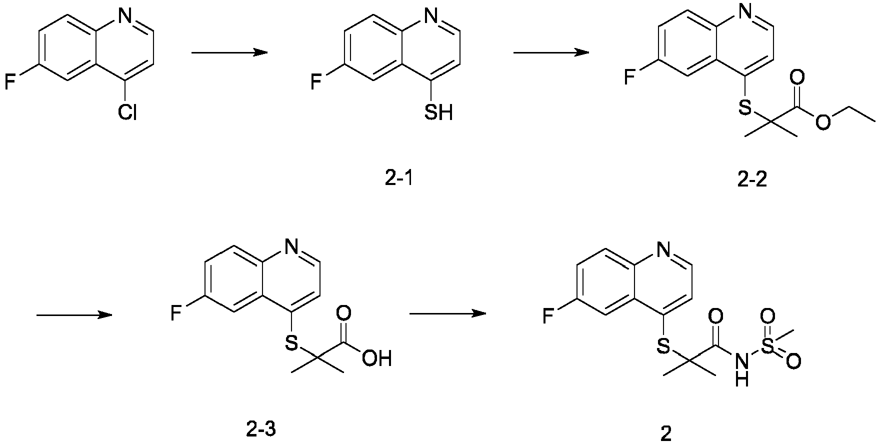

Synthesis of an intermediate 2-1: 4-chloro-6-fluoro-quinoline (5.0 g, 28 mmol), sodium sulfide (13.5 g, 56 mmol), and 25 mL of DMF were added into a 100 mL three-neck flask, and stirred at 100°C for 2 h under the protection of nitrogen gas. After cooling to room temperature, the reaction solution was poured into 100 mL of water, and extracted with dichloromethane (50 mL *3), and then an organic phase was removed. The pH of the water phase was regulated to 5-6 with concentrated hydrochloric acid; and stirring was performed for 1 h to separate out a yellow solid. Vacuum filtration was performed, and vacuum drying was performed at 50°C to obtain 3.8g of a yellow solid, and the yield was 77%.

Synthesis of intermediate 2-2: The intermediate 2-1 (3.4 g, 19 mmol), ethyl bromomethylpropionate (5.5 g, 28 mmol), cesium carbonate (12.3 g, 38 mmol) and 30 mL of DMF were added into a 100 mL three-neck flask, and stirred at room temperature for 2 h. The reaction solution was poured into 100 mL of ice water, and extracted with DCM (50 mL *3), and the organic phase was combined. Then washing was performed with a saturated salt solution (100 mL *2), drying was performed with sodium sulfide, and the organic phase was concentrated. Column chromatography (300-400 meshes, and eluted with petroleum ether) was performed on the crude product to obtain 5.2 g of a colorless oily substance; and the yield was 93%.

Synthesis of intermediate 2-3: The intermediate 2-2 (5 g, 17 mmol), sodium hydroxide (1.38 g, 34 mmol), 30 mL of methanol and 5 mL of water were added into a 100 mL three-neck flask, and stirred at 60°C for 2 h. After the reaction was finished, 20 mL of water was added for dilution, and concentrating was performed to remove methanol. The pH of the water phase was regulated to 1-2 with concentrated hydrochloric acid to obtain a yellow solid, and the yellow solid was subjected to vacuum drying at 50°C to obtain 3.1 g of the crude product. The yield was 69%.

Synthesis of compound 2: The intermediate 2-3 (0.6 g, 2.3 mmol), methyl sulfonamide (0.65 g, 6.8 mmol), HATU (1.1 g, 2.9 mmol), DIPEA (0.9 g, 6.9 mmol) and 7 mL of DMF were added into a reaction bottle, and stirred at room temperature for 24 h. The reaction solution was poured into 50 mL of ice water, extracted with EA (50 mL *3), washed with saturated salt water (50 mL *2), dried over sodium sulfide, and then concentrated to obtain a crude product. High-pressure preparation separation (reversed-phase column C-18, mobile phase: acetonitrile and water) was performed on the crude product. Concentrating was performed to remove acetonitrile. Then freeze-drying was performed to obtain 0.27g of a white solid. The yield was 35%. Mass spectrum: 343.0 (M+H⁺).

Compound 2: Mass spectrum: 343, 344 (M+H⁺); ¹H NMR (400MHz, DMSO-d⁶) δ (ppm): 8.88 (d, J = 4.8Hz, 1H), 8.20-8.10 (m, 2H), 7.90-7.70 (m, 2H), 7.60 (d, J = 4.8Hz, 1H), 3.18 (s, 3H), 1.59 (s, 6H).

### Example 3

The synthesis method of compound 10 is the same as the synthesis method of compound 1. The synthetic route is as follows:

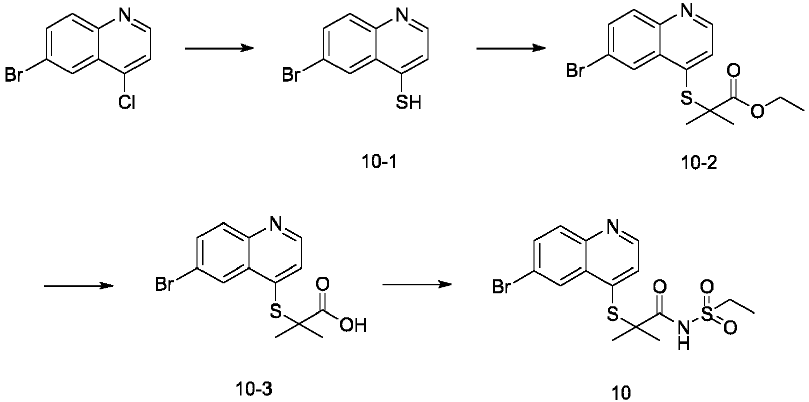

Synthesis of intermediate 10-1: 4-chloro-6-bromo-quinoline (5.0 g, 21 mmol), sodium sulfide (10.0 g, 41 mmol), and 25 mL of DMF were added into a 100 mL three-neck flask, and stirred at 100°C for 2 h under the protection of nitrogen gas. After cooling to room temperature, the reaction solution was poured into 100 mL of water, and extracted with dichloromethane (50 mL *3), and then an organic phase was removed. The pH of the water phase was regulated to 5-6 with concentrated hydrochloric acid; and stirring was performed for 1 h to separate out a yellow solid. Vacuum filtration was performed, and vacuum drying was performed at 50°C to obtain 4.1g of a yellow solid, and the yield was 82%.

Synthesis of intermediate 10-2: The intermediate 10-1 (4.5 g, 19 mmol), ethyl bromomethylpropionate (4.0 g, 20 mmol), cesium carbonate (12.3 g, 38 mmol) and 30 mL of DMF were added into a 100 mL three-neck flask, and stirred at room temperature for 2 h. The reaction solution was poured into 100 mL of ice water, and extracted with DCM (50 mL *3), and the organic phase was combined. Then washing was performed with a saturated salt solution (100 mL *2), drying was performed with sodium sulfide, and the organic phase was concentrated. Column chromatography (300-400 meshes, and eluted with petroleum ether) was performed on the crude product to obtain 6.0g of a colorless oily substance; and the yield was 91%.

Synthesis of intermediate 10-3: The intermediate 10-2 (5 g, 14 mmol), sodium hydroxide (1.38 g, 34 mmol), 30 mL of methanol and 5 mL of water were added into a 100 mL three-neck flask, and stirred at 60°C for 2 h. After the reaction was finished, 20 mL of water was added for dilution, and concentrating was performed to remove methanol. The pH of the water phase was regulated to 1-2 with concentrated hydrochloric acid to obtain a yellow solid, and the yellow solid was subjected to vacuum drying at 50°C to obtain 3.3g of the crude product. The yield was 72%.

Synthesis of compound 10: The intermediate 10-3 (0.75 g, 2.3 mmol), methyl sulfonamide (0.65 g, 6.8 mmol), HATU (1.1 g, 2.9 mmol), DIPEA (0.9 g, 6.9 mmol) and 7 mL of DMF were added into a reaction bottle, and stirred at room temperature for 24 h. The reaction solution was poured into 50 mL of ice water, extracted with EA (50 mL *3), washed with saturated salt water (50 mL *2), dried over sodium sulfide, and then concentrated to obtain a crude product. High-pressure preparation separation (reversed-phase column C-18, mobile phase: acetonitrile and water) was performed on the crude product. Concentrating was performed to remove acetonitrile. Then freeze-drying was performed to obtain 0.23 g of a white solid. The yield was 25%. Mass spectrum: 417 (M+H⁺).

Compound 10: Mass spectrum: 417, 419 (M+H⁺); ¹H NMR (400MHz, DMSO-d⁶) δ (ppm): 8.89 (d, *J* = 4.8Hz, 1H), 8.52 (d, *J* = 2.0Hz, 1H), 8.10-7.90 (m, 2H), 7.53 (d, *J* = 4.8Hz, 1H), 3.40-3.30 (m, 2H), 1.62 (s, 6H), 1.14 (t, *J* = 7.2, 3H).

### Example 4

The synthesis method of compound 4 is the same as the synthesis method of compound 1. The synthetic route is as follows:

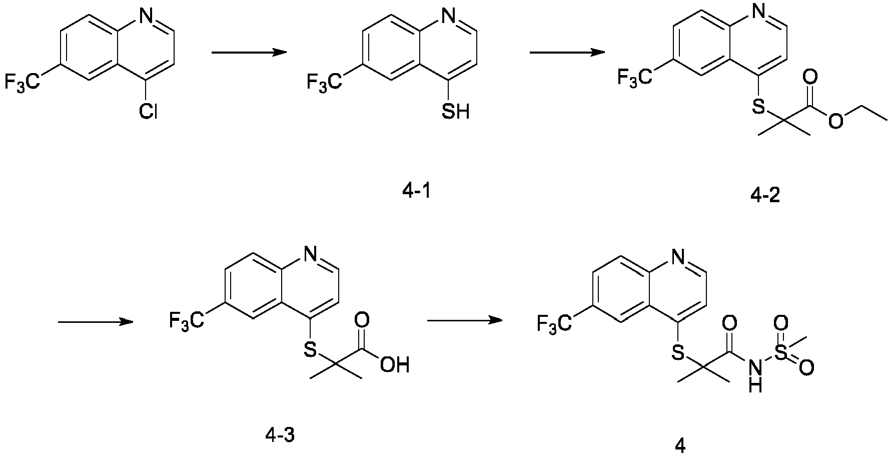

Synthesis of intermediate 4-1: 4-chloro-6-trifluoromethylquinoline (5.0 g, 21 mmol), sodium sulfide (10.4 g, 44 mmol), and 25 mL of DMF were added into a 100 mL three-neck flask, and stirred at 100°C for 2 h under the protection of nitrogen gas. After cooling to room temperature, the reaction solution was poured into 100 mL of water, and extracted with dichloromethane (50 mL *3), and then an organic phase was removed. The pH of the water phase was regulated to 5-6 with concentrated hydrochloric acid; and stirring was performed for 1 h to separate out a yellow solid. Vacuum filtration was performed, and vacuum drying was performed at 50°C to obtain 4.5g of a yellow solid, and the yield was 90%.

Synthesis of intermediate 4-2: The intermediate 4-1 (3.3 g, 14 mmol), ethyl bromomethylpropionate (4.0 g, 20 mmol), cesium carbonate (12.3 g, 38 mmol) and 30 mL of DMF were added into a 100 mL three-neck flask, and stirred at room temperature for 2 h. The reaction solution was poured into 100 mL of ice water, and extracted with DCM (50 mL *3), and the organic phase was combined. Then washing was performed with a saturated salt solution (100 mL *2), drying was performed with sodium sulfide, and the organic phase was concentrated. Column chromatography (300-400 meshes, and eluted with petroleum ether) was performed on the crude product to obtain 4.1 g of a colorless oily substance; and the yield was 82%.

Synthesis of intermediate 4-3: The intermediate 4-2 (4 g, 12 mmol), sodium hydroxide (1.38 g, 34 mmol), 30 mL of methanol and 5 mL of water were added into a 100 mL three-neck flask, and stirred at 60°C for 2 h. After the reaction was finished, 20 mL of water was added for dilution, and concentrating was performed to remove methanol. The pH of the water phase was regulated to 1-2 with concentrated hydrochloric acid to obtain a yellow solid, and the yellow solid was subjected to vacuum drying at 50°C to obtain 2.7 g of the crude product. The yield was 73%.

Synthesis of compound 4: The intermediate 4-3 (0.72g, 2.3 mmol), methyl sulfonamide (0.65 g, 6.8 mmol), HATU (1.1 g, 2.9 mmol), DIPEA (0.9 g, 6.9 mmol) and 7 mL of DMF were added into a reaction bottle, and stirred at room temperature for 24 h. The reaction solution was poured into 50 mL of ice water, extracted with EA (50 mL *3), washed with saturated salt water (50 mL *2), dried over sodium sulfide, and then concentrated to obtain a crude product. High-pressure preparation separation (reversed-phase column C-18, mobile phase: acetonitrile and water) was performed on the crude product. Concentrating was performed to remove acetonitrile. Then freeze-drying was performed to obtain 0.77 g of a white solid. The yield was 86%. Mass spectrum: 393 (M+H⁺).

Compound 4: Mass spectrum: 393, 395 (M+H⁺); ¹H NMR (400MHz, CDCl₃) δ (ppm): 8.91 (d, *J* = 5.2Hz, 1H), 8.55 (s, 1H), 8.33 (d, *J* = 8.8Hz, 1H), 8.00 (dd, *J* = 8.8Hz, *J* = 1.6Hz, 1H), 7.45 (d, *J* = 5.2Hz, 1H), 3.31 (s, 3H), 1.78 (s, 6H).

### Example 5

The synthesis method of compound 18 is the same as the synthesis method of compound 10. The synthetic route is as follows:

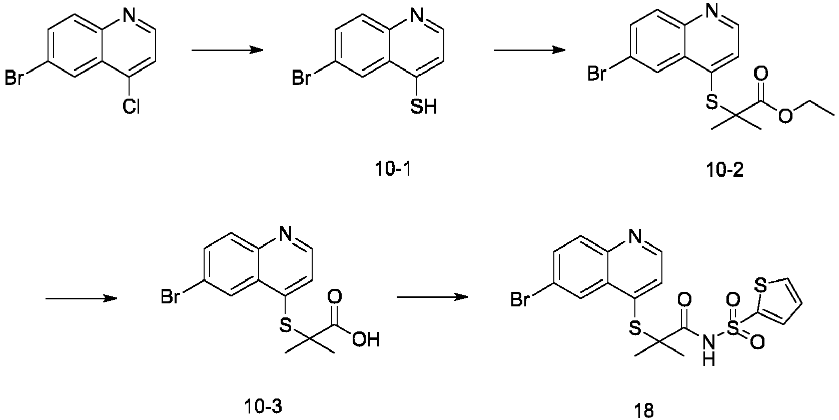

Synthesis of compound 18: The intermediate 10-3 (0.75 g, 2.3 mmol), thiophenesulfonamide (1.1 g, 6.8 mmol), HATU (1.1 g, 2.9 mmol), DIPEA (0.9 g, 6.9 mmol) and 7 mL of DMF were added into a reaction bottle, and stirred at room temperature for 24 h. The reaction solution was poured into 50 mL of ice water, extracted with EA (50 mL *3), washed with saturated salt water (50 mL *2), dried over sodium sulfide, and then concentrated to obtain a crude product. High-pressure preparation separation (reversed-phase column C-18, mobile phase: acetonitrile and water) was performed on the crude product. Concentrating was performed to remove acetonitrile. Then freeze-drying was performed to obtain 0.34g of a white solid. The yield was 32%. Mass spectrum: 471 (M+H⁺).

Compound 18: Mass spectrum: 471 (M+H⁺); ¹H NMR (400MHz, DMSO-d⁶) δ (ppm): 8.57 (d, *J* = 4.8Hz, 1H), 8.50 (d, *J* = 2.0Hz, 1H), 8.05 (dd, *J* = 4.8Hz, *J* = 2.0Hz, 1H), 7.98 (s, 1H), 7.96 (d, *J* = 2.0Hz, 1H), 7.77 (dd, *J* = 4.0Hz, *J* = 1.6Hz, 1H), 7.22 (dd, *J* = 4.8Hz, *J* = 4.0Hz, 1H), 6.87 (d, *J* = 4.8Hz, 1H), 1.54 (s, 6H).

### Activity test for target compounds

### I. Experimental purpose

It was to test the *in vitro* inhibitory activity (IC50) of the compound on hURAT1.

### II. Experimental materials

### 2.1 To-be-tested compounds:

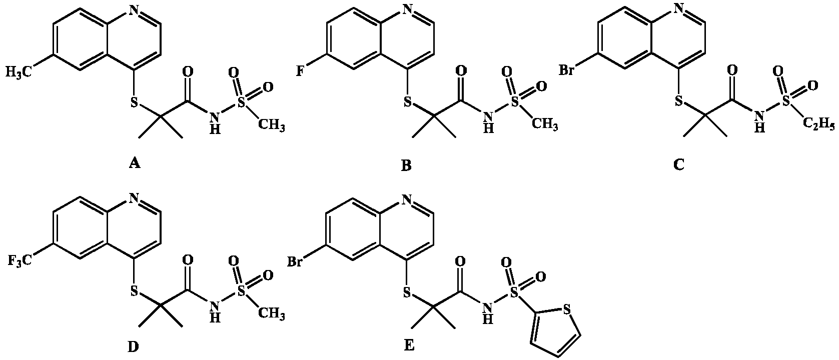

### 2.2 HEK-293T cell lines stably expressing hURAT was independently constructed by Shanghai ChemPartner Co., Ltd.

### 2.3 The following materials were purchased by Shanghai ChemPartner Co., Ltd.:

| Name of reagent | Article number | Manufacturer |
|---|---|---|
| Fetal bovine serum | 10099141 | Invitrogen |
| DMEM culture medium | 10564 | Invitrogen |
| Trypsin | 25200056 | Invitrogen |
| G418 | ant-gn-5 | Invitrogen |
| Phosphate buffer solution | 14190250 | Invitrogen |
| ¹⁴C-uric acid | ARC0513-250UCI | ARC |
| Dimethyl sulfoxide | D2650 | Sigma |
| 15 mL centrifuge tube | 07030115 | Greiner |
| 50 mL centrifuge tube | 352098 | BD Falcon |
| Penicillin- Streptomycin | 15070-063 | Invitrogen |
| Benzbromarone | 3562-84-3 | J&K Scientific |
| Sodium D-gluconate | 527-07-1 | Aladdin |
| Potassium D-gluconate | 299-27-4 | Aladdin |
| Calcium D-gluconate | 299-28-5 | Aladdin |

### III. Experimental methods

### 3.1 Preparation of experimental reagents

| | |
|---|---|
| Cl- free HBSS buffer solution | 125 mM sodium gluconate |
| Cl- free HBSS buffer solution | 4.8 mM potassium gluconate |
| Cl- free HBSS buffer solution | 1.3 mM calcium gluconate |
| Cl- free HBSS buffer solution | 1.2 mM KH₂PO₄ |
| Cl- free HBSS buffer solution | 1.2 mM MgSO4 |
| Cl- free HBSS buffer solution | 5.6 mM glucose |
| Cl- free HBSS buffer solution | 25 mM HEPES (pH=7.4) |
| Lysate | 100 mM NaOH |

### 3.2 Cell culture and inoculation

(1) HEK-293T cell strains stably expressing hURAT1 were cultured. The culture medium included: a DMEM culture medium+10% of fetal calf serum+500 µg/ml G418+1% of P/S.
(2) When the cells grew to 80% full, the culture medium was removed, PBS was added to clean the cells once, and then pancreatin-EDTA was added for digestion. The culture medium was added when the cells were subjected to wall removal, and subjected to blowing and beating to make the cells fall off. The cells were centrifuged and collected. The culture medium was added, and subjected to blowing and beating to form cell suspension.
(3) The cell density was adjusted to be 7×10⁵/ml, then the cells were inoculated into a 96-well cell culture plate with white wall and transparent bottom according to the amount of 100 µL/well, and cultured for 12-24 h.

### 3.3 Preparation of compounds

(1) The compound was prepared into a mother solution with a concentration of 20 mM by DMSO, the mother solution was diluted to reach a concentration of 1 mM by DMSO, and then the solution was added into 96 wells.
(2) Quality control compounds were additionally arranged on the 96-well plate, so as to obtain a 100 x compound plate.
(3) The solutions in corresponding wells of another 96-well plate were diluted 50 times by the Cl-free HBSS buffer solution, so as to obtain a 2 x compound plate (plate 2).
(4) A buffer solution containing 0.1 µCi/ml 14C-uric acid at a density of 30 µL/well and a 2 x diluted compound at a density of 30 µL/well into a new 96-well plate, so as to obtain a 1 x compound plate (plate 1) for later use.

### 3.4 Absorption of ¹⁴C-uric acid in cells stably expressing hURAT1

(1) An absorption test may be performed after cells in the 96-well plate were cultured and adhered to the wall.
(2) The cells were washed once by the preheated buffer solution at a density of 200 µL/well.
(3) The cells in each well were completely absorbed, and then the corresponding compounds and 0.1 µCi/ml 14C-uric acid solution were immediately added at a density of 50 µL/well.
(4) The plate added with the compounds was incubated in an incubator at 37°C for 5 min.
(5) 150 µL of ice-cold buffer solution was immediately added into each well to stop absorption. Each well was washed for three times with the buffer solution. (Note: it is needed to avoid cell shedding in the washing process as much as possible.)
(6) Lysate was added into all wells at a density of 50 µL/well, and the wells were placed on an oscillator to be oscillated at a speed of 900 rpm for 5 min.
(7) Scintillation solution Microsint40 was added into all wells at a density of 150 µL/well, and the wells were oscillated at a speed of 900 rpm for 5 min.
(8) Finally, a microporous plate was transferred to a MicroBeta2 (Produced by PerkinElmer Company) instrument to measure the radioactivity.
(9) Data was analyzed, and each compound IC50 was calculated by GraphPad Prism 5 software.

### IV. Experimental results

The experimental results are as represented by Table 1. The activity test showed that the compounds A-E had good inhibitory activity and were worthy of further research.

**Table 1: IC50 data of compounds A-E inhibiting the activity of hURAT1**

| No. | BOTTOM | TOP | HILLSLOPE | IC50 (M) |
|---|---|---|---|---|
| Benzbromarone¹ | 6.1 | 99.1 | 1.20 | 5.85E-07 |
| A¹ | -0.2 | 74.6 | 2.2 | 3.15E-06 |
| Verinuard² | 0.00 | 96.94 | 0.75 | 5.45E-08 |
| B² | 2.79 | 111.10 | 0.69 | 2.79E-06 |
| C² | 7.49 | 95.14 | 0.89 | 1.18E-06 |
| D² | -18.87 | 106.60 | 0.48 | 8.04E-08 |
| E² | -0.97 | 90.15 | 1.26 | 3.42E-06 |

| | | | | |
|---|---|---|---|---|
| Note: 1 represents the hURAT1 activity inhibition test on the plate1; and 2 represents the hURAT1 activity inhibition test on the plate2. | | | | |

The above-mentioned examples of the present application are only examples for illustrating the present application, and are not intended to limit the embodiment of the present application. For those of ordinary skill in the art, other different forms of changes and modifications can be made based on the above description. It is not possible to exhaustively list all possible embodiments here. Any obvious changes or modifications derived from the technical solutions of the present application are still within the scope of protection of the present application.

## Claims

1. A quinoline mercaptoacetate sulfonamide derivative, comprising a structure as represented by the following formula: 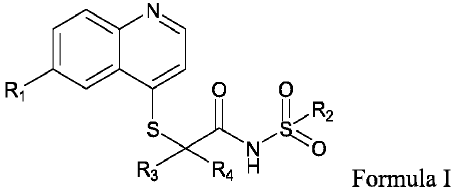
R₁ and R₂ are each independently selected from hydrogen, halogen, cyano, nitro, substituted or unsubstituted C1-C6 linear alkyl, substituted or unsubstituted C3-C7 cycloalkyl, substituted or unsubstituted C3-C7 heterocycloalkyl, and substituted or unsubstituted C4-C12 heterocyclic aryl; heteroatoms are selected from one or more of oxygen, sulfur, and nitrogen; and substituents are selected from one or more of halogen, cyano, nitro, alkoxy, alkyl, haloalkyl, hydroxyalkyl, alkenyl, alkynyl, cycloalkyl, heterocycloalkyl, aryl, and heterocyclic aryl;
R₃ and R₄ are each independently selected from a hydrogen atom, C1-C6 alkyl or cycloalkyl; or R₃ and R₄ form a C3-C6 ring.

2. The compound according to claim 1, wherein in the structure as represented by Formula I:
R₁ and R₂ are selected from hydrogen, halogen, cyano, nitro, C1-C3 linear alkyl, C1-C4 branched alkyl, C3-C6 cycloalkyl, C3-C4 substituted cycloalkyl, phenyl, substituted phenyl, thiophene, and C5-C6 heterocyclic aryl; the substituent is selected from halogen, cyano, methyl, ethyl, n-propyl, isopropyl, cyclopropyl, and cyclobutyl; the heteroatoms in the heterocyclic aryl are selected from one or more of oxygen, sulfur, and nitrogen; and
R₃ and R₄ are each independently selected from a hydrogen atom, C1-C3 alkyl or cycloalkyl; or R₃ and R₄ form a C2-C4 ring.

3. The compound according to claim 1 or 2, wherein
R₁ is selected from hydrogen, halogen, cyano, nitro, C1-C3 linear alkyl, C1-C4 branched alkyl, and C3-C6 cycloalkyl;
R₂ is selected from C1-C3 linear alkyl, C1-C4 branched alkyl, C3-C6 cycloalkyl, C3-C4 substituted cycloalkyl, phenyl, substituted phenyl, thiophene, and C5-C6 heterocyclic aryl; the substituent is selected from halogen, cyano, methyl, ethyl, n-propyl, isopropyl, cyclopropyl, and cyclobutyl; and the heteroatoms in the heterocyclic aryl are selected from one or more of oxygen, sulfur, and nitrogen; and
R₃ and R₄ are each independently selected from a hydrogen atom, methyl, and ethyl.

4. The compound according to any one of claims 1 to 3, wherein R₁ is methyl, fluorine, bromine, or trifluoromethyl; R₂ is methyl, ethyl, thiophene, or cyclopropyl; and R₃ and R₄ are each independently selected from methyl.

5. The compound according to any one of claims 1 to 4, wherein the compound with the structure as represented by Formula I comprises the following specific compounds:

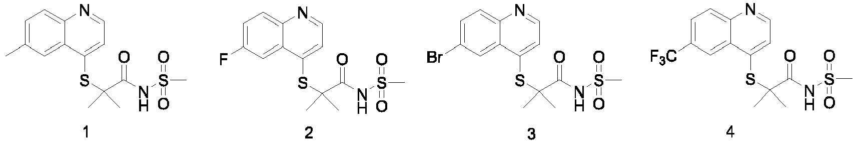

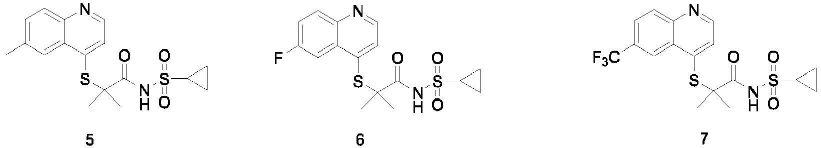

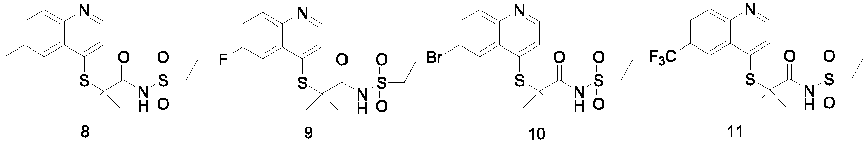

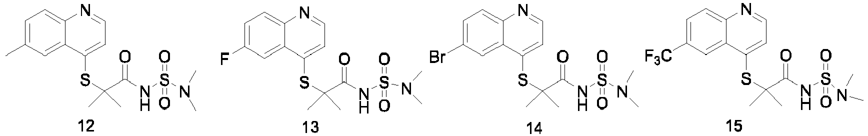

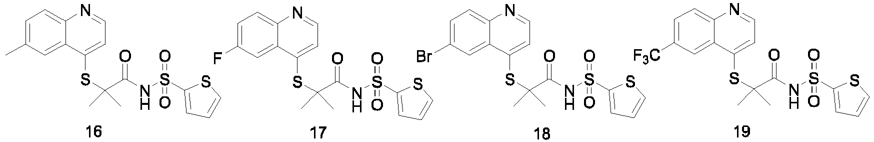

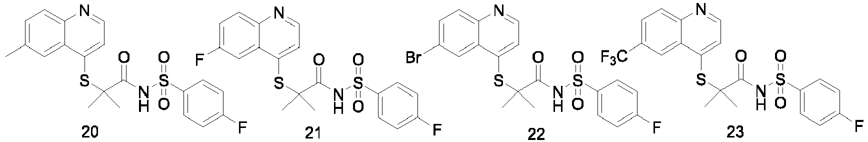

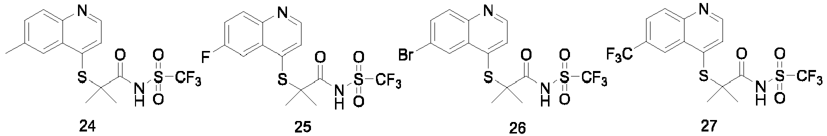

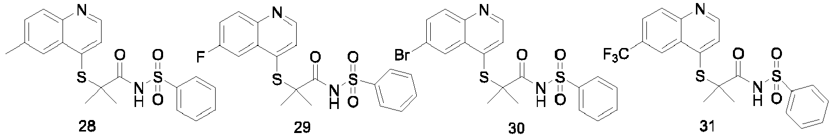

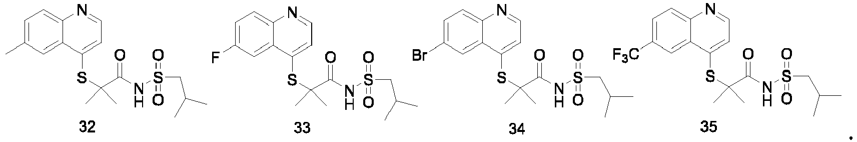

6. An intermediate for preparing the compound according to any one of claims 1 to 5, comprising a structure as represented by Formula II, 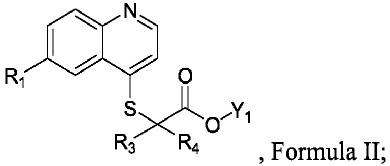
in the structure of the compound as represented by Formula II,
R₁ is R₁ in Formula I, and Y₁ is methyl or ethyl; and
R₃ and R₄ are R₃ and R₄ in Formula I.

7. A method for preparing the intermediate according to claim 6, comprising:
(1) performing first contact on a compound as represented by Formula III and a sulfide salt in the presence of a first solvent and an inert atmosphere, so as to obtain a compound as represented by Formula IV; and
(2) performing second contact on the compound as represented by Formula IV and a compound as represented by Formula V in the presence of the first solvent and carbonate salt; 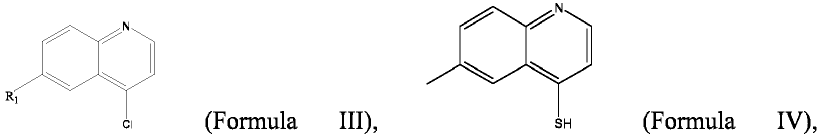 and 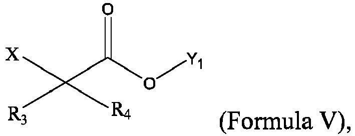
R₁ is R₁ in Formula I, and Y₁ is methyl or ethyl;
R₃ and R₄ are R₃ and R₄ in Formula I; and
X is halogen.

8. A method for preparing the compound according to any one of claims 1 to 5, comprising: sequentially performing hydrolysis reaction and sulfonamide reaction on the compound as represented by Formula II according to claim 6 to obtain the compound.

9. The method according to claim 8, wherein the hydrolysis reaction process comprises: performing third contact on a solution containing the compound as represented by Formula II and an alkaline aqueous solution, and performing heating reflux.

10. The method according to claim 8, wherein the sulfonamide reaction process comprises: in the presence of a second solvent, performing fourth contact on the compound as represented by Formula II-2 and a sulfonamide compound.

11. The method according to any one of claims 8 to 10, comprising the following reactions:

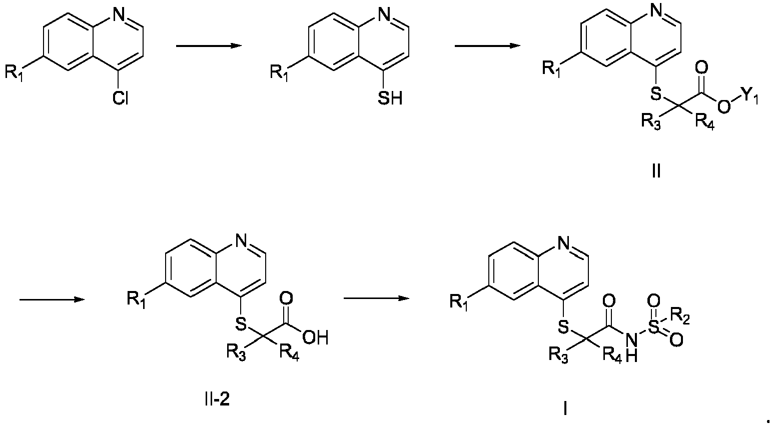

12. A pharmaceutical derivative or a formulation of the compound as represented by Formula I according to any one of claims 1 to 5, comprising a pharmaceutically acceptable salt, a composition, a solvate, a hydrate, and a pharmaceutically acceptable prodrug.

13. A pharmaceutical derivative or a formulation of the compound as represented by Formula II according to claim 6, comprising a pharmaceutically acceptable salt, a composition, a solvate, a hydrate, and a pharmaceutically acceptable prodrug.

14. Use of the compound as represented by Formula I according to any one of claims 1 to 5, the pharmaceutical derivative or the formulation of the compound as represented by Formula I according to claim 12, the compound as represented by Formula II according to claim 6, and the pharmaceutical derivative or the formulation of the compound as represented by Formula II according to claim 13 in preparation of a medicine for regulating the level of uric acid and/or treating gout-related indications.

15. The use according to claim 14, wherein the related indications comprise, but are not limited to, hyperuricemia, gout, gouty arthritis, inflammatory arthritis, nephropathy, nephrolithiasis, arthritis, urate crystal deposition in joints, urolithiasis, urate crystal deposition in renal parenchyma, gout attack, tophaceous gout, or a combination thereof.
